# EUROPEAN PATENT APPLICATION

(11) **EP 4 686 451 A1**
(43) Date of publication of application: **04.02.2026**
(21) Application number: 25192823.0
(22) Date of filing: 30.07.2025
(51) Int. Cl.: A61B 17/00, A61F 2/00

(54) **APPARATUS FOR TREATMENT OF A MUSCLE HOLE**

(30) Priority: 02.08.2024 IT 202400018145
(71) Applicant: Amato, Giuseppe, 90144 Palermo (IT)
(72) Inventor: Amato, Giuseppe, 90144 Palermo (IT)
(74) Representative: Varini, Marcello

(57) **Abstract**

An apparatus **(100)** for the treatment of a muscle hole **(105)** is provided. The apparatus **(100)** comprises: - a flexible plate member **(130)** having a main surface; - a shaft member **(120)** inserted across the flexible plate member **(130)** so as the main surface extends substantially perpendicularly to the shaft member **(120)** when the apparatus **(100)** is in an implanted configuration, said main surface covering said muscle hole **(105)** when the apparatus **(100)** is in the implanted configuration; - a flexible extremity member **(140)** coupled to the shaft member **(120)** and configured to be inserted into said muscle hole **(105)** when the apparatus **(100)** is in the implanted configuration, the flexible extremity member **(140)** comprising a plurality of elastic pushing elements **(140(p))** arranged to surround a portion **(SP)** of the shaft member **(120),** said portion **(SP)** of the shaft member **(120)** having a first portion end **(SP(1))** distal to the flexible plate member **(130)** and a second portion end **(SP(2))** proximal to the flexible plate member **(130),** each elastic pushing element **(140(p))** being biased to push against lateral edges of the muscle hole **(105)** at least perpendicularly to the shaft member **(120),** so as to keep the apparatus **(100)** locked in the muscle hole **(105),** when the apparatus **(100)** is in the implanted configuration. Each elastic pushing element **(140(p))** has a first element end **(140(1))** coupled to said first portion end **(SP(1))** and a second element end **(140(2))** coupled to said second portion end **(SP(2)),** the elastic pushing element **(140(p))** being bent to have a central section thereof between the first element end **(140(1))** and the second element end **(140(2))** that is spaced apart from the shaft member **(120)** when the apparatus **(100)** is in the implanted configuration so as to define an empty space between the elastic pushing element **(140(p))** and said portion **(SP)** of the shaft member **(120).**

## Description

### Technical field

The present invention generally relates to the field of the apparatuses for treating defects in muscular walls. More particularly, the present invention relates to an apparatus for repairing abdominal wall or pelvic floor hernias.

### Background art

The background of the present disclosure is hereinafter introduced with the discussion of devices and techniques relating to its context.

A hernia is a condition occurring when an organ or tissue protrudes through a weak point - *e.g.*, a hole - in the surrounding muscular walls. This typically manifests as a bulge in the abdominal or groin area. Hernias can vary in severity, from causing minimal discomfort to leading to significant pain and complications.

Different types of hernias exist, including abdominal wall hernias - such as inguinal, incisional or Spigelian hernias - and pelvic floor hernias - such as obturator hernias.

Conventional surgical techniques for treating hernia, including open and laparoscopic approaches, involve the reinforcement of the muscular wall through the deployment of a flat mesh over the muscle hole and the fixation of the mesh through stitches or tacks.

### Summary

The Applicant has realized that while the conventional surgical techniques for managing hernia have been widely adopted, they are associated with several drawbacks.

Open hernia repair typically requires a large skin incision and broad dissection of tissue layers, leading to increased postoperative pain and a longer recovery period. Moreover, the placement of flat mesh relies on sutures or staples to secure it in place. These fixation methods can cause tissue damage, bleeding, hematoma, increase the risk of infection, and contribute to chronic pain.

Laparoscopic hernia repair, although less invasive, also presents challenges. While it typically involves smaller incisions, the placement of flat mesh can be technically demanding, requires broad tissue dissection with traumatic instruments and enhanced surgical skills. Moreover, similar to open techniques, sutures or staples are used to secure the mesh, which can lead to complications.

Additionally, after its implantation the pressure of the abdominal content exerts tension upon the fixation tools that may detach leaving the mesh unanchored. This can cause over time mesh dislodgement, potentially compromising the repair and leading to hernia recurrence.

Further, conventional flat meshes are static and passive, unable to adapt to the dynamic movements of the abdominal wall and pelvic floor.

Moreover, the abovementioned approaches do not allow to completely solve the hernia issue, as the deployment of a mesh of this type is not able to heal the roots of hernia disease which is degenerative. Actually, known flat meshes induce a foreign body reaction with long lasting inflammatory response leading to uncontrolled ingrowth of stiff scar tissue that can enfold sensitive nerves running close to the surgical site. This occurrence may trigger the so called chronic pain syndrome caused by exuberant fibrotic development leading to compression of nervous structures.

In view of the above, the Applicant realized that there exists a need for improved apparatus and methods for treating defects in muscular walls, particularly abdominal wall and pelvic floor hernias. Such advancements should aim to address the limitations of current techniques, minimize complications, enhance patient recovery, and provide durable and long-lasting hernia repair solutions.

One or more aspects of the present invention are set out in the independent claims, with advantageous features of the same invention that are indicated in the dependent claims, whose wording is enclosed herein verbatim by reference (with any advantageous feature being provided with reference to a specific aspect of the present invention that applies mutatis mutandis to any other aspect thereof).

An aspect of the present invention relates to an apparatus for the treatment of a muscle hole.

The apparatus comprises a flexible plate member having a main surface.

The apparatus comprises a shaft member inserted across the flexible plate member so as the main surface extends substantially perpendicularly to the shaft member when the apparatus is in an implanted configuration.

Said main surface covers said muscle hole when the apparatus is in the implanted configuration.

The apparatus comprises a flexible extremity member coupled to the shaft member.

The flexible extremity member is configured to be inserted into said muscle hole when the apparatus is in the implanted configuration.

The flexible extremity member comprises a plurality of elastic pushing elements arranged to surround a portion of the shaft member.

Said portion of the shaft member has a first portion end distal to the flexible plate member and a second portion end proximal to the flexible plate member.

Each elastic pushing element is biased to push against lateral edges of the muscle hole at least perpendicularly to the shaft member, so as to keep the apparatus locked in the muscle hole, when the apparatus is in the implanted configuration.

Each elastic pushing element has a first element end coupled to said first portion end and a second element end coupled to said second portion end.

Each elastic pushing element is bent to have a central section thereof between the first element end and the second element end that is spaced apart from the shaft member when the apparatus is in the implanted configuration so as to define an empty space between the elastic pushing element and said portion of the shaft member.

After the apparatus is implanted, the muscle hole is therefore protected by the plate member, and at the same time the apparatus is kept locked into the muscle hole without the need of fixation means like stitches, tacks, sutures or staples or the like.

The apparatus can be advantageously implanted easily very quickly, without requiring any surgical dissection or tissue trauma of structures surrounding the hole.

The elastic properties of the pushing elements and their configuration allow the implanted apparatus to dynamically move together in tune with the muscle wall, following its movement in all the directions, without dislodging.

Moreover, once the apparatus is implanted, the elastic mechanical interaction between the elastic pushing elements and the lateral edges of the muscle hole attracts tissue growth factors inside the volume delimited by the elastic pushing elements when the apparatus is in the implanted configuration.

According to an embodiment of the present invention, each elastic pushing element is arranged to have the central section thereof that is spaced apart from the ones of the adjacent pushing elements so as to define empty spaces therebetween when the apparatus is in the implanted configuration.

This peculiar arrangement of the pushing elements advantageously provides a scaffold for the regrowth of muscles, nerves and vessels in said empty spaces once the apparatus is implanted.

According to an embodiment of the present invention, the flexible plate member comprises a hole through which the shaft member is inserted, allowing the flexible plate member sliding along the shaft member.

According to an embodiment of the present invention, a movement of the flexible plate member toward said first portion end presses the flexible extremity member and moves said second element ends toward said first portion end, thereby increasing the bending of said elastic pushing elements.

In this way, the extension of the flexible extremity member perpendicularly to the shaft member may be easily tuned to fit the features of the muscle hole.

According to an embodiment of the present invention, the shaft member comprises one or more stopper elements.

According to an embodiment of the present invention, each stopper element is configured to prevent a further sliding of the flexible plate member away from said first portion end when the flexible plate member is located between the first portion end and said stopper element and at the same time is in contact with said flexible plate member.

In this way, once the flexible plate member reached a stopper element corresponding to a desired configuration of the flexible extremity member, undesired relative movement between the flexible plate member and the shaft member capable of modifying said desired configuration is advantageously prevented.

According to an embodiment of the present invention, the flexible extremity member comprises a joint element joining together the second element ends of the elastic pushing elements.

According to an embodiment of the present invention, the joint element comprises a hole.

According to an embodiment of the present invention, the shaft member is inserted through said hole of the joint element, so that said joint element is able to slide along the shaft member.

According to an embodiment of the present invention, said joint element is configured to be in contact with said flexible plate member, so that a movement of the flexible plate member toward said first portion end presses the joint element toward said first portion end.

According to an embodiment of the present invention, said first portion end corresponds to an end of the shaft member.

According to an embodiment of the present invention, the apparatus further comprises a clamp element configured to fix the first element ends of the elastic pushing elements to said first portion end.

According to an embodiment of the present invention, the apparatus comprises a thermoplastic elastomer. Other materials may be used in addition or in the place of a thermoplastic elastomer, such as Polyester, Polyurethane, Polypropylene or Polyethylene or combination thereof.

According to an embodiment of the present invention, said muscle hole is a hole located in the abdominal wall or in the pelvic floor.

### Brief description of the annexed drawings

These and other features and advantages of the present invention will appear more clearly by reading the following detailed description of exemplary and non-limitative embodiments thereof. For its better intelligibility, the following description should be read making reference to the attached drawing, wherein:
**Figure 1** shows an apparatus for repairing a muscle hole according to an embodiment of the present invention in an implanted configuration, deployed inside a muscle hole to be repaired;
**Figure 2A** is a perspective view of the apparatus of **Figure 1** in an implanted configuration;
**Figure 2B** is a side view of the apparatus of **Figure 1** in an implanted configuration;
**Figure 2C** is a bottom view of the apparatus of **Figure 1** in an implanted configuration;
**Figure 2D** is a perspective section view of the apparatus of **Figure 1** in an implanted configuration;
**Figure 3** is a side view of the apparatus of **Figure 1** in a delivery configuration;
**Figure 4A** is a top view of a plate member of the apparatus of **Figure 1** according to an embodiment of the present invention;
**Figure 4B** is a perspective view of an extremity member of the apparatus of **Figure 1** according to an embodiment of the present invention;
**Figures 5A** and **5B** are samples of healthy tissues grown in the place of a muscle hole stimulated by the use of the apparatus of **Figure 1** according to an embodiment of the present invention.

### Detailed description of exemplary embodiments

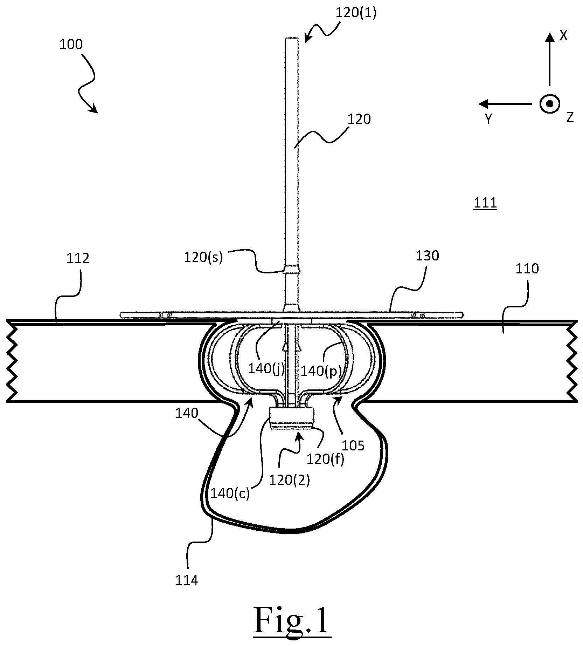

With reference to the drawings, **Figures 1****,** **2A-2D** and **3** show an apparatus **100** for the treatment of a muscle hole (hereinafter, simply referred to as "the apparatus" for the sake of brevity) according to embodiments of the present invention.

Unless otherwise specified, directional terminology (for example, upper, lower, top, bottom, lateral, side, front, rear, longitudinal, horizontal, transverse, vertical) associated with the apparatus **100** and components thereof will be used in connection with their orientation in the figures. On this regard, in the following, directional terminology is referred to mutually orthogonal reference directions **X, Y, and Z.**

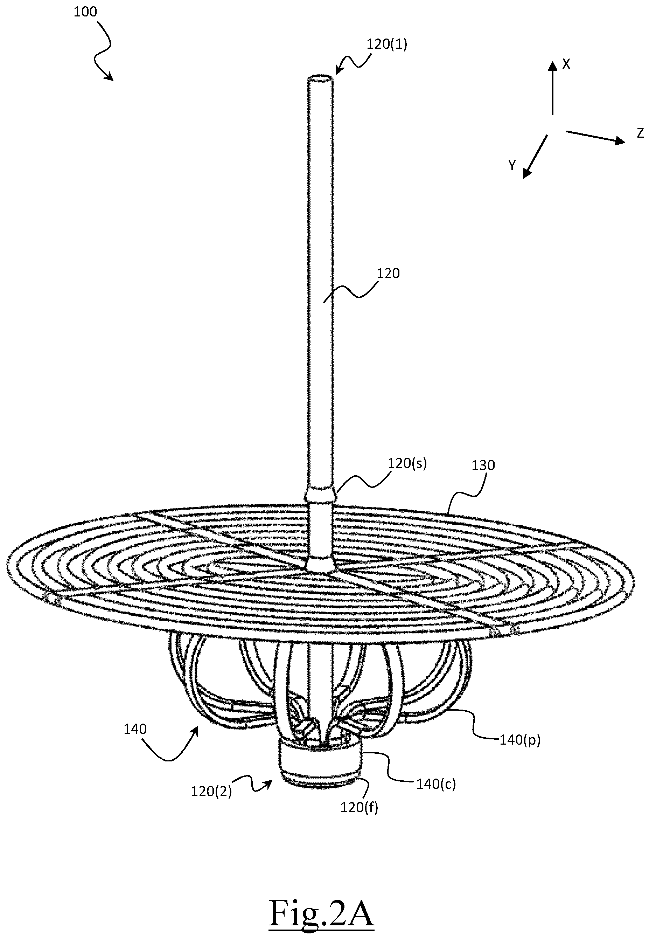

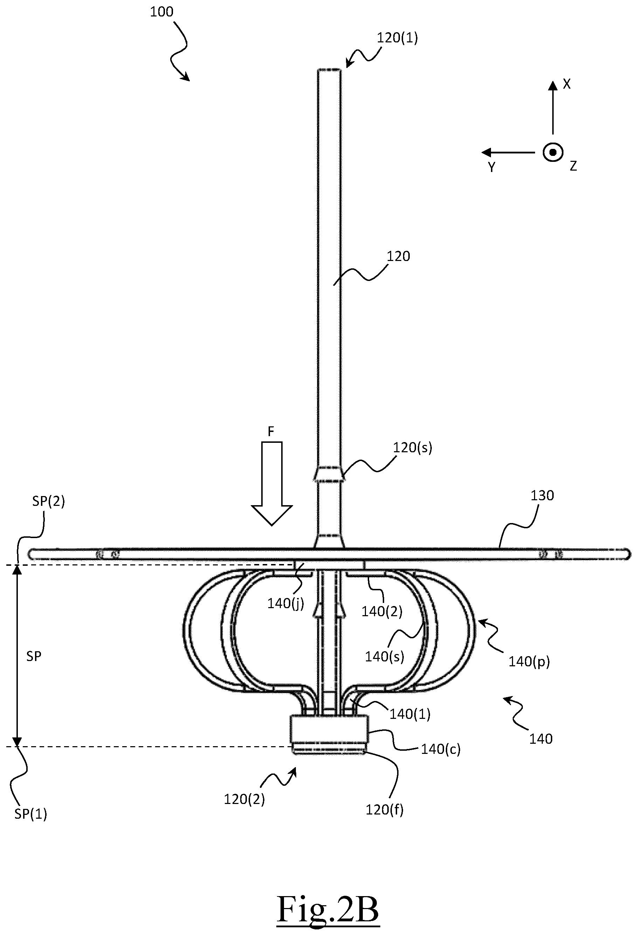

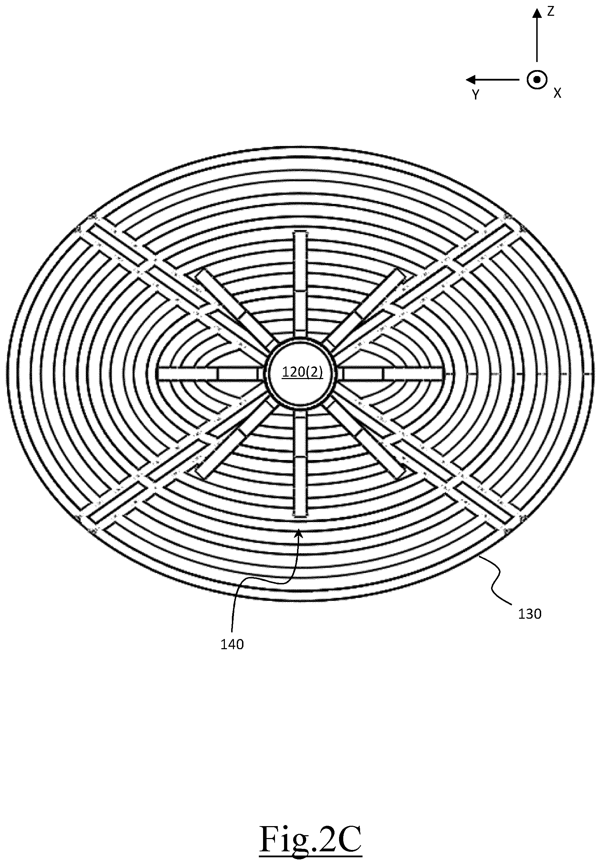

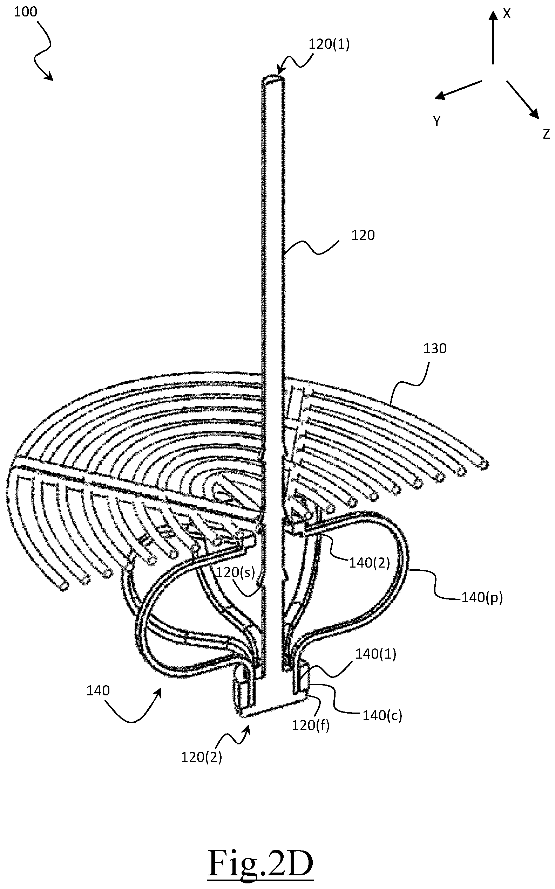

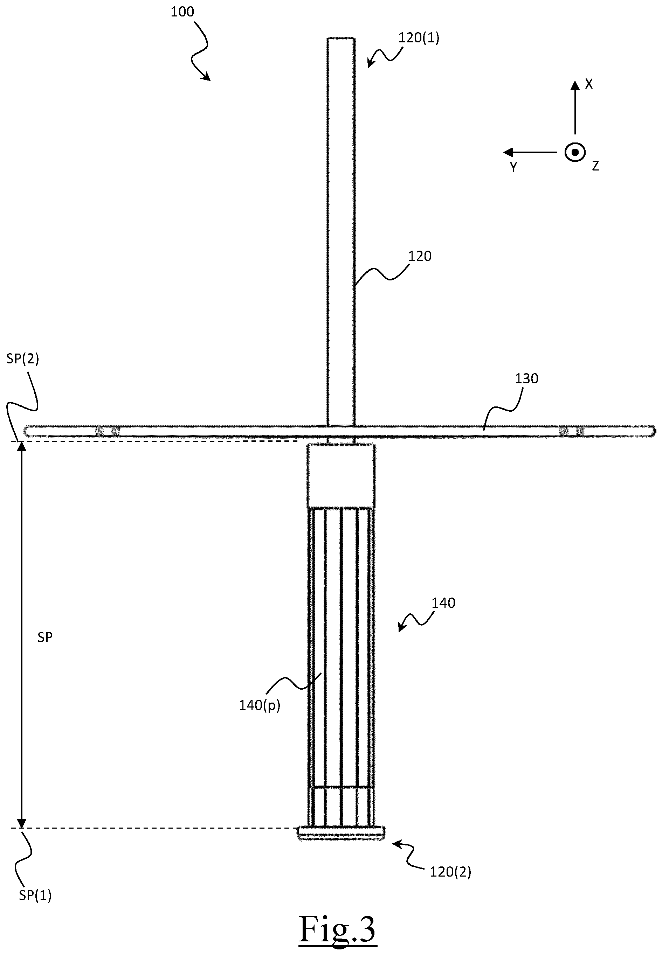

**Figure 1** shows the apparatus **100** in an implanted configuration, deployed inside a muscle hole to be repaired. **Figure 2A** is a perspective view of the apparatus **100** in an implanted configuration, **Figure 2B** is a side view of the apparatus 100 in an implanted configuration taken from a plane view parallel to the directions **X** and **Y,** **Figure 2C** is a bottom view of the apparatus **100** in an implanted configuration taken from a plane view parallel to the directions **Z** and **Y,** and **Figure 2D** is a perspective view of the apparatus **100** in an implanted configuration sectioned along a section plane parallel to the directions **X** and **Y.** **Figure 3** is a side view of the apparatus **100** in a delivery configuration, *i.e.,* in a configuration used during the delivery of the apparatus **100** in the muscle hole, before the deployment of the apparatus, taken from a plane view parallel to the directions **X** and **Y.**

The muscle hole to be treated by the apparatus **100** according to the embodiments of the present invention, identified in **Figure 1** with reference **105,** is an opening in a muscle wall, identified in **Figure 1** with reference **110,** caused by a hernia condition, for example developed over time due to strain or pressure exerted by the pushing of an organ or tissue located in the abdominal cavity, identified in **Figure 1** with reference **111.** The muscle wall **110** may for example correspond to the abdominal wall or the pelvic floor. In this case, the muscle wall **110** is lined internally by the peritoneum, indicated in **Figure 1** with reference **112.** Following the formation of the muscle hole **105,** the peritoneum **112** forms a saccular extroflection **114** that crosses the muscle hole heading outside the muscle wall.

According to an embodiment of the present invention, the apparatus **100** comprises a shaft member **120,** a flexible plate member **130** and a flexible extremity member **140.**

As will be described in details in the following, according to an embodiment of the present invention:
- the shaft member **120** is configured to support the plate member **130** and the extremity member **140,**
- the plate member **130** is configured to shield the muscle hole **105** *(e.g.,* from pushing organs or tissue), and
- the extremity member **140** is configured to carry out the double task of keeping the apparatus **100** locked inside the muscle hole **105** when in an implanted configuration and provide a scaffold for the regrowth of muscles, nerves and vessels in the place of the muscle hole **105.**

According to an embodiment of the present invention, the shaft member 120 vertically extends along direction **X** from a first end **120(1)** to a second end **120(2),** with the apparatus **100** that is configured to be inserted in the muscle hole **105** oriented with the second end **120(2)** that is facing toward the muscle hole **105** (as will be described in the following).

Preferably, the shaft member **120** has a circular or oval section (along directions **Y** and **Z),** although similar considerations may be applied to shaft members having a different section, such as for example prismatic shaft members having a polygonal section.

According to an embodiment of the present invention, the section (along directions **Y** and **Z)** of the shaft member **120** may for example have a diameter ranging from 2 to 8 mm.

According to an embodiment of the present invention, the second end **120(2)** of the shaft member **120** has larger section (*e.g.*, having a larger diameter) along directions **Y** and **Z** compared to the rest of the shaft member **120.**

According to an embodiment of the present invention, the section (along directions **Y** and **Z)** of the second end **120(2)** of the shaft member **120** may for example have a diameter ranging from 3 to 12 mm.

According to an embodiment of the present invention, the second end **120(2)** is also provided with a flange member **120(f)** having different dimensions, protruding perpendicularly to the direction **X** from the second end **120(2)** itself. In the exemplary embodiment illustrated in the figures, the flange member **120(f)** is a circular flange member, however the concepts of the present invention can be directly applied to flange members of different shapes, such as having a polygonal shape.

According to an embodiment of the present invention, the flange member **120(f)** may for example protrude (perpendicularly to direction **X)** from the second end **120(2)** of the shaft member **120** by an amount ranging from 2 to 6 mm.

According to an embodiment of the present invention, the thickness (along direction **X)** of the second end **120(2)** with the larger section may be selected based on the features and sizes of the abdominal cavity **111** and of the tissues surrounding it.

According to an embodiment of the present invention, the thickness (along direction **X)** of the second end **120(2)** with the larger section may for example range from 3 to 8 mm.

According to an embodiment of the present invention, the shaft member **120** comprises one or more (three, in the exemplary embodiment illustrated in the figures) stopper elements **120(s)** protruding from the shaft member **120** and configured to interact with the plate member **130,** as will be described in the following.

According to an embodiment of the present invention, when two or more stopper elements **120(s)** are provided (like in the case of the three stopper elements **120(s)** of the exemplary embodiment illustrated in the figures), said stopper elements **120(s)** are located at different positions of the shaft member **120** along direction **X.**

According to an embodiment of the present invention, each stopper element **120(s)** has the shape of a truncated cone, having a symmetry axis coaxial to the axis of the shaft member **120,** a smaller base having the same size of the shaft member **120** section, and orientated to exhibit the larger base proximal to the second end **120(2)** and the smaller base distal to the second end **120(2).** Similar considerations in case the stopper elements **120(s)** have a different shape, provided that they comprise a portion protruding at least partially perpendicularly to direction **X** from the shaft member **120.**

According to an embodiment of the present invention, the protruding portion of each stopper element **120(s)** may for example protrude (perpendicularly to direction **X)** from the shaft member **120** by an amount ranging from 0,5 to 3 mm.

According to an embodiment of the present invention, the shaft member **120** comprises a medical grade biocompatible material. For example, according to an embodiment of the present invention, the shaft member **120** may be made of an injection molded or 3D printed medical grade Thermoplastic Elastomer (TPE), Polyester, Polyurethane, Polypropylene or Polyethylene or combination of these materials. Other materials suitable for permanent implantation within the human body may be also contemplated.

According to an embodiment of the present invention, the plate member **130** has a main surface that extends along directions **Y** and **Z.** In the exemplary embodiment illustrated in the figures, the plate member **130** has the shape of a flat oval plate. However, the concepts of the present invention may be applied in case the plate member has a different shape, such as for example a disc shape, a rectangular shape, a squared shape, an ogival shape and so on.

According to an embodiment of the present invention, the main surface may for example be equal to 4-10 cm x 10-15 cm.

According to an embodiment of the present invention, the plate member **130** comprises a plurality of spaced apart and interconnected wire or thread elements forming a mesh-like structure in 3D fashion extending along directions **Y** and **Z** and defining the main surface of the plate member **130.**

According to an embodiment of the present invention, each thread element may have a rectangular, circular, oval, squared section, or a combination thereof.

According to an embodiment of the present invention, each thread element may have a thickness of ranging from 0,1 to 3 mm.

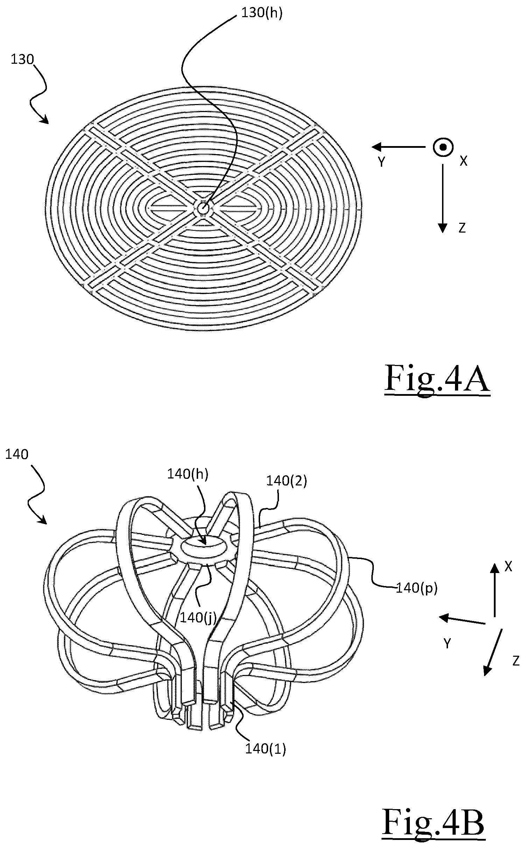

As visible in **Figure 4A****,** showing a top view of the plate member **130** detached from the shaft member **120,** according to an embodiment of the present invention, the plate member **130** is provided with a *(e.g.,* central) through-hole **130(h)** through which the shaft member **120** is inserted so that the main surface of the plate member **130** extends substantially perpendicular to the (axis of the) shaft member **120.**

According to an embodiment of the present invention, the through-hole **130(h)** in the plate member **130** allows the plate member **130** to slide (along the direction **X)** along the shaft member **120.**

According to an embodiment of the present invention, the size (along directions **Y** and **Z)** of the through-hole **130(h)** is sufficiently large to allow the sliding of the plate member **130** along the shaft member **120,** but is smaller than the size (along directions **Y** and **Z)** of the second end **120(2).**

According to an embodiment of the present invention, the diameter of the through-hole **130(h)** may for example be larger than the diameter of the section (along directions **Y** and **Z)** of the shaft member **120** by an amount ranging from 0,4 to 1 mm.

Moreover, according to an embodiment of the present invention, the the size (along directions **Y** and **Z)** of the through-hole **130(h)** of the plate member **130** is (*e.g.,* only slightly) smaller than the size (along directions **Y** and **Z)** of the the stopper elements **120(s)** of the shaft member **120.** By making reference to the exemplary embodiment of the invention illustrated in the figures, in which each stopper element **120(s)** has the shape of a a truncated cone, the size (along directions **Y** and **Z)** of the through-hole **130(h)** is *(e.g.,* only slightly) smaller than the size (along directions **Y** and **Z)** of the larger base of the truncated cone.

According to an embodiment of the present invention, the plate member **130** comprises a medical grade biocompatible material exhibiting elastic proprieties, such as for example an elastomer. For example, according to an embodiment of the present invention, the plate member **130** may be made of an injection molded or 3D printed medical grade TPE, Polyester, Polyurethane, Polypropylene or Polyethylene or combination of these materials. Other materials exhibiting elastic proprieties and suitable for permanent implantation within the human body may be also contemplated.

Thanks to the elastic proprieties of the material of the plate member **130,** and thanks to its mesh-like structure, the plate member **130** is flexible and can be easily squeezed, so as it can be suitably introduced in a trocar channel of adequate dimension to be delivered during laparoscopic operations into the abdominal cavity **111.**

According to an embodiment of the present invention, at least the main surface of the plate member **130** that is configured to face toward the inside of the abdominal cavity **111** when the apparatus **100** is in the implanted configuration may be coated or provided with an anti-adhesion layer of any kind of material. In this way, adhesion bridles to viscera located inside the abdominal cavity **111** may be further prevented.

According to embodiments of the present invention, the anti-adhesion layer may cover only the main surface of the plate member **130** that is configured to face toward the inside of the abdominal cavity **111,** or may also extend to cover also (portions or of the entire) opposite surface *(e.g.,* the surface that is configured to face toward the muscle wall **110).**

According to an embodiment of the present invention, said anti-adhesion layer may be in the form of or comprising a biologic film of any chemical composition.

According to another embodiment of the present invention, said anti-adhesion layer may comprise an anti-adhesion tissue that is glued or molded at least onto the main surface of the plate member **130.** For example, said anti-adhesion tissue may comprise an anti-adhesive synthetic fabric such as for example expanded polytetrafluoroethylene (ePTFE/Teflon) or other materials already employed as anti-adhesive barrier for medical use.

When the shaft member **120** is inserted inside the through-hole **130(h)** of the plate member **130** from the first end **120(1)** and the plate member **130** is made to slide along the shaft member **120** toward the second end **120(2),** the plate member **130** is able to go beyond the stopper elements **120(s).** Indeed, thanks to the peculiar form of the stopper elements **120(s)** and the flexibility of the the plate member **130,** by exerting a pressure on the plate member **130** toward the second end **120(2)** when the plate member **130** reaches a stop member **120(s),** a temporary deformation (*i.e.,* an expansion) of the through-hole **130(h)** of the plate member **130** is promoted by the sliding along the slanted side surface of the *(e.g.,* conical) stop member **120(s),** causing the through-hole **130(h)** of the stopper element **130** take a size sufficient for crossing the stopper element **120(s).**

The plate member **130** is instead prevented to go beyond a stopper element **120(s)** when moving in the opposite direction, *i.e.,* when sliding along the shaft member **120** from the second end **120(2)** to the first end **120(1)** because of the presence of the protruding step between the larger base of the stopper element 120(s) and the shaft member **120.**

According to an embodiment of the present invention, the extremity member **140** comprises a plurality of elastic pushing elements **140(p)** arranged to surround a portion **SP** (see **Figures 2B** and **3****)** of the shaft member **120.** In the exemplary and not limitative embodiment of the present invention illustrated in the figures, the extremity member **140** comprises eight pushing elements **140(p),** however the concepts of the present invention can be applied to a different number of pushing elements **140(p),** such as for example three, four, six, ten, twelves or even more.

According to an embodiment of the present invention, the portion **SP** of the shaft member **120** surrounded by the pushing elements **140(p)** of the extremity member **140** is located between the second end **120(2)** of the shaft member **120** and the plate member **130** (when the plate member **130** is coupled to the shaft member **120** through the through-hole).

According to an embodiment of the present invention, the portion **SP** of the shaft member **120** surrounded by the elastic pushing elements **140(p)** of the extremity member **140** extends from a first portion end **SP(1)** distal to the plate member **130** to a second portion **SP(2)** proximal to the plate member **130** (when the plate member **130** is coupled to the shaft member **120** through the through-hole).

According to an embodiment of the present invention, the first portion end **SP(1)** corresponds to the second end **120(2)** of the shaft member **120.** However, the concepts of the present invention can be applied also in case the first portion end **SP(1)** is spaced apart from the second end **120(2)** of the shaft member **120.**

According to an embodiment of the present invention, the second portion end **SP(2)** is located under the plate member **130.**

According to an embodiment of the present invention, each pushing element **140(p)** is in the shape of a rod having a first element end **140(1)** and a second element end **140(2)** coupled to the shaft member **120** (visible in **Figures 2B** and **2D****).**

According to an embodiment of the present invention, the length of each pushing element **140(p)** from the first element end **140(1)** to the second element end **140(2)** may be set based on the kind/size of muscle hole **105** to be repaired, and may for example span from less than 0.5 cm to 20 cm or even more.

In the exemplary embodiment of the invention illustrated in the figures, the pushing elements **140(p)** have the shape of rods having a rectangular section, however similar considerations apply in case the section is different, such as for example circular, oval, squared or a combination thereof.

According to an embodiment of the present invention, the section of each pushing element **140(p)** may be set based on the kind/size of muscle hole **105** to be repaired, and may for example span from 0.1 mm x 0,1 mm to 5 mm x 5 mm or more.

According to an embodiment of the present invention, when the apparatus **100** is in the delivery configuration (see **Figure 3****),** the pushing elements **140(p)** are not bent longitudinally, and extend substantially parallel to the shaft member **120** along direction **X.** According to an embodiment of the present invention, the first element ends **140(1)** of the pushing elements **140(p)** are fixed to the shaft member **120,** for example by means of a clamp element **140(c).** In the exemplary embodiment of the present invention, the clamp element **140(c)** is a ring element in contact with the flange member **120(f)** of the shaft member **120** and configured to clamp the first element ends **140(1)** against the second end **120(2).**

According to an embodiment of the present invention, the extremity member **140** further comprises a joint element **140(j)** joining together the second element ends **140(2)** of the pushing elements **140(p).**

As visible in **Figure 4B****,** showing a perspective view of the extremity member **140** detached from the shaft member **120,** according to an embodiment of the present invention, the joint element **140(j)** of the extremity member **140** is provided with a through hole **140(h)** to allow the shaft member **120** be inserted therethrough, so that the joint element **140(j)** is able to slide along the shaft member **120.**

According to an embodiment of the present invention, each pushing element **140(p)** is flexible. Since the first element ends **140(1)** of the pushing elements **140(p)** are fixed to the shaft member **120,** while the second element ends **140(2)** of the pushing elements **140(p)** are joined together through a joint element **140(j)** which is able to slide along the shaft member **120** (along the direction **X),** the flexibility of the pushing elements **140(p)** allow them to be bent outwards (perpendicularly to direction **X)** if a force **F** parallel to direction **X** and directed toward the second end **120(2)** is applied to their second element ends **140(2)** (see **Figure 2B****).**

According to an embodiment of the present invention, if the plate member **130** is pushed along the shaft member **120** against the joint element **140(j)** toward the second end **120(2)** of the shaft member **120,** the joint element **140(j)** slides toward the second end **120(2),** causing the pushing elements **140(p)** bend outward (perpendicularly to direction **X)** so as a central section of the pushing elements **140(p)** between the first and second element ends **140(1), 140(2)** becomes spaced apart (perpendicularly to direction **X)** from the shaft member **120.** This condition is illustrated in **Figures 1****,** **2A-2D****,** in which the apparatus **100** is in an implanted configuration.

According to an embodiment of the present invention, the more the plate member **130** (and therefore, the joint element **140(j))** is pushed toward the second end **120(2)** of the shaft member **120,** the shorter the portion **SP** of the shaft member **120** surrounded by the elastic pushing elements **140(p)** of the extremity member **140,** and the larger the space between the central sections of the pushing elements **140(p)** and the shaft member **120.**

According to an embodiment of the present invention, the plate member **130** (and therefore, the joint element **140(j))** may be also pushed toward the second end **120(2)** of the shaft member **120** to an extent such to cause the elastic pushing elements **140(p)** to fold beyond the second end **120(2)** so that the second end **120(2)** as well is surrounded by the elastic pushing elements **140(p)** of the extremity member **140.** In this configuration, the second end **120(2)** of the shaft member **120** results to be "embedded" within the extremity member **140,** with at least a portion of each pushing element **140(p)** that is located at a height (along direction **X)** that is below the second end **120(2)).** This configuration may be for example reached when the plate member **130** is pushed until crossing a stopper element **120(s)** provided at a distance from the second end **120(2)** of the shaft member **120** that is so small to allow a direct contact between the joint element **140(j)** and the second end **120(2).**

According to an embodiment of the present invention, when the apparatus **100** is in the delivery configuration (see **Figure 3****),** adjacent pushing elements **140(p)** are very close to each other, and the space there between is substantially small.

According to an embodiment of the present invention, when the plate member **130** is pushed against the joint element **140(j)** and the joint element **140(j)** slides toward the second end **120(2),** the bending of the pushing elements **140(p)** also causes each central section of each pushing element **140(p)** be spaced apart from the central sections of the adjacent pushing elements **140(p)** so as to define empty spaces therebetween, as illustrated in **Figures 1****,** **2A-2D****.**

According to an embodiment of the present invention, also the extremity member **140** comprises a biocompatible medical grade material exhibiting flexible and/or elastic proprieties, such as for example an elastomer. For example, according to an embodiment of the present invention, the extremity member **140** may be made of an injection molded or 3D printed medical grade TPE, Polyester, Polyurethane, Polypropylene or Polyethylene or a combination of these materials. Other materials exhibiting elastic proprieties and suitable for permanent implantation within the human body may be also contemplated.

The apparatus **100** according to the embodiments of the present invention described above has been designed to be mainly delivered laparoscopically by a surgeon. However, the apparatus **100** according to the embodiments of the present invention may be also be delivered with robotic approach, through open surgery and/or through different approaches, such as for example by means of Natural Orifice Translumenal Endoscopic Surgery (NOTES).

By making for example reference to the laparoscopic surgical approach, in order to repair a muscle hole in a muscular wall located in the abdominal cavity **111,** the apparatus **100** may be introduced into the abdominal cavity **111** in the following way.

Firstly, the apparatus **100** is configured in the delivery configuration, with the pushing elements **140(p)** of the extremity member **140** that are not bent, and extend substantially parallel to the shaft member **120** along direction **X,** and the plate member **130** that is loosely fitted on the shaft member **120** (as illustrated in **Figure 3****).**

Then, the grip of a laparoscopic forceps is inserted in a, *e.g.,* metallic, tube, and the apparatus **100** is firmly grasped from the first end **120(1)** of the shaft member **120** by the grasping jaws of the forceps.

With this configuration, the apparatus **100** is thus delivered into the abdominal cavity **111** through a trocar channel of suitable dimension, and guided toward the muscle hole to be repaired.

The extremity member **140** of the apparatus **100** is introduced into the muscle hole to be repaired. Since the apparatus is in the delivery configuration, the size, perpendicularly to the shaft member **120,** of the extremity member **140** is sufficiently small to easily penetrate into the muscle hole to be repaired. For example, the size of the extremity member **140** perpendicular to the shaft member **120** is smaller than the diameter of the muscle hole.

Then, with a combined maneuver, the tube is pushed against the plate member **130** to cause the plate member **130** slide along the shaft member **120** in the direction toward the second end **120(2),** while at the same time the forceps pulls backwards in the opposite direction the shaft member **120.**

During its movement, the plate member **130** pushes the extremity member **140,** so that the pushing elements **140(p)** start to bend and protrude perpendicularly to the shaft member **120.** Thus, the (central portions of) pushing elements **140(p)** engage the lateral edges of the muscle hole to be repaired.

The plate member **130** is made to slide with respect to the shaft member **120** until a selected stopper element **120(s)** is crossed. If more than one stopper element **120(s)** is provided on the shaft member **120,** this selection is carried out by taking into account the actual size of the muscle hole to be repaired. Indeed, as already described above, by varying the position of the plate member **130** along the shaft member **120,** and therefore by varying the length of the portion **SP** of the shaft member **120** that is actually surrounded by the elastic pushing elements **140(p)** of the extremity member **140,** it is possible to control the extension of the bent elastic pushing elements **140(p)** perpendicularly to the shaft member **120.** The plate member **130** is particularly slid until the crossing of a stopper element **120(s)** corresponding to an extension of the bent elastic pushing elements **140(p)** fits the diameter of the muscle hole, so as the lateral edges of the muscle hole to be repaired are properly expanded and supported by the pushing elements **140(p)** in a way similar to the one of a stent device.

In this implanted configuration of the apparatus **100,** corresponding to the one illustrated in **Figure 1****:**
- the muscle hole **105** is covered by the plate member **130,** which rests on the surfaces of the peritoneum **112** covering the muscle wall **110** surrounding the muscle hole **105;**
- the extremity member **140** is firmly fitted inside the muscle hole **105,** with the elastic pushing elements **140(p)** that are biased to push against the lateral edges of the muscle hole **105** at least perpendicularly to the shaft member **120.**

To ensure a more accurate and/or stable placement, a simple forceps guided adjustment of the plate member **130** can be made (the plate member **130** may be rotated with respect to the shaft member **120** without risks of dislodgments along direction **X** because the size of the through-hole **130(h)** is larger than the diameter of the shaft member **120),** and/or a stress test may be conducted by pulling the apparatus **100** from the first end **120(1)** of the shaft member **120** to assess the stability of the extremity member **140** inside the muscle hole **105.**

At this point, the redundant portion of the shaft member **120,** *i.e.,* the one extending from the first end **120(1)** of the shaft member **120** to the stopper element **120(s)** contacting the plate member **130,** is removed by excision through scissors.

The apparatus **100** according to the embodiments of the present invention can be implanted easily very quickly, without requiring any surgical dissection or tissue trauma of structures surrounding the hole, as is usually made with the known, conventional meshes.

After the apparatus **100** is implanted, the muscle hole **105** is protected by the plate member **130** from pushing organs or tissue that may cause a new hole in the neighboring areas, if unprotected. At the same time, the apparatus **100** is kept locked into the muscle hole **150** by the lateral forces exerted by the pushing elements **140(p)** against the lateral edges of the muscle hole **105,** without the need of fixation means like stitches, tacks, sutures or staples or the like. The pain discomfort typical of the use of fixation means on the neighboring muscular structure is therefore entirely avoided.

The elastic properties of the pushing elements **140(p),** and their shape and configuration allow the implanted apparatus **100** to dynamically move together in tune with the muscle wall **110,** following its movement in all the directions, without dislodging.

Moreover, once the apparatus **100** is implanted, the elastic mechanical interaction between the pushing elements **140(p)** of the extremity member **140** and the lateral edges of the muscle hole **105** (which are both elastic structures) attracts tissue growth factors inside the volume delimited by the pushing elements **140(p)** when the apparatus **100** is in the implanted configuration. Said tissue growth factors foster the development of newly formed muscles, nerves and vessels that regenerate the muscular barrier, thus ultimately filling the muscle hole **105.** In response to the natural cyclic compression and relaxation of the muscle wall **110,** the pushing elements **140(p)** of the extremity member **140** are accordingly cyclically compressed and released, transmitting to the side edges of the muscle hole **105** a periodic pressure difference (in all the directions) capable of promoting the re-establishment of the muscle wall and related components thanks the regenerative effects exerted by specific tissue growth factors.

Unlike conventional flat meshes, which are uncapable of promoting tissue regeneration, and instead cause uncontrolled stiff scar tissue development that may enfold neighboring nerves causing chronic pain, the apparatus **100** according to the embodiments of the invention obliterates in a fixation free fashion the muscle hole **105,** and thanks to its dynamic responsivity to muscle movements induces the growth of healthy connective tissue, muscles, nerves and vessels in the empty spaces between the pushing elements **140(p)** of the extremity member **140** and the surrounded portion **SP** of the shaft member **120.** From this point of view, the extremity member **140** operates as a scaffold triggering the growth of new connective tissue, muscles, nerves and vessels, *i.e.,* the components of the muscular barrier that typically delimit the abdominal cavity **111.**

The occurrence of the growth factors and the corresponding growth of healthy muscles, nerves and vessels in the place of the muscle hole **105** stimulated by the use of the apparatus **100** has been histologically verified by the Applicant by analyzing sections of samples extracted from tissue grown in muscle holes purchased in the abdominal walls of several test pigs after the implantation in these muscle holes of the apparatus **100** according to the embodiments of the present invention.

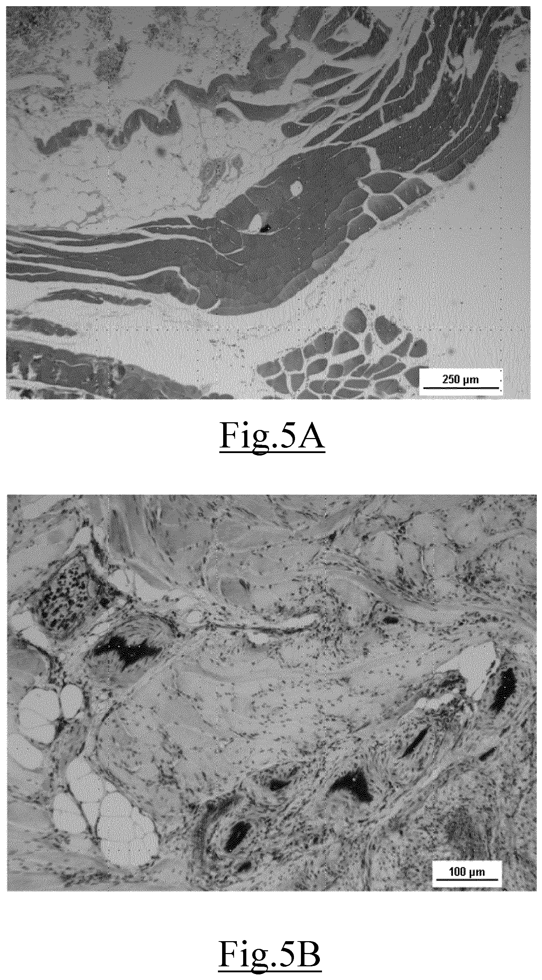

Two of these samples are illustrated in **Figures 5A** and **5B****.** As can be observed from these samples, no inflammation towards the components of the apparatus **100** is visible, while healthy muscles and vessels are visible in **Figure 5A****,** and healthy nerves are visible in **Figure 5B****.**

During the test carried out by the Applicant, evidences of several growth factors have been observed, such as:
- VEGF, CD31, and SMA in relation to vascular development;
- NGF supporting the development of newly formed myocytes and the presence of mature muscular elements;
- NGFRp75 indicating neurogenic development and the presence of mature neural elements.

Naturally, in order to satisfy local and specific requirements, a person skilled in the art may apply to the disclosure described above many logical and/or physical modifications and alterations. More specifically, although the disclosure has been described with a certain degree of particularity with reference to preferred embodiments thereof, it should be understood that various omissions, substitutions and changes in the form and details as well as other embodiments are possible. In particular, different embodiments of the disclosure may even be practiced without the specific details (such as the numeric examples) set forth in the preceding description for providing a more thorough understanding thereof; on the contrary, well-known features may have been omitted or simplified in order not to obscure the description with unnecessary particulars.

## Claims

1. Apparatus **(100)** for the treatment of a muscle hole **(105),** comprising:
- a flexible plate member **(130)** having a main surface;
- a shaft member **(120)** inserted across the flexible plate member **(130)** so as the main surface extends substantially perpendicularly to the shaft member **(120)** when the apparatus **(100)** is in an implanted configuration, said main surface covering said muscle hole **(105)** when the apparatus **(100)** is in the implanted configuration;
- a flexible extremity member **(140)** coupled to the shaft member **(120)** and configured to be inserted into said muscle hole **(105)** when the apparatus **(100)** is in the implanted configuration, the flexible extremity member **(140)** comprising a plurality of elastic pushing elements **(140(p))** arranged to surround a portion **(SP)** of the shaft member **(120),** said portion **(SP)** of the shaft member **(120)** having a first portion end **(SP(1))** distal to the flexible plate member **(130)** and a second portion end (**SP(2)**) proximal to the flexible plate member **(130),** each elastic pushing element **(140(p))** being biased to push against lateral edges of the muscle hole **(105)** at least perpendicularly to the shaft member **(120),** so as to keep the apparatus **(100)** locked in the muscle hole **(105),** when the apparatus **(100)** is in the implanted configuration, wherein:
- each elastic pushing element **(140(p))** has a first element end **(140(1))** coupled to said first portion end **(SP(1))** and a second element end **(140(2))** coupled to said second portion end **(SP(2)),** the elastic pushing element **(140(p))** being bent to have a central section thereof between the first element end **(140(1))** and the second element end **(140(2))** that is spaced apart from the shaft member **(120)** when the apparatus **(100)** is in the implanted configuration so as to define an empty space between the elastic pushing element **(140(p))** and said portion **(SP)** of the shaft member **(120).**

2. The apparatus **(100)** of claim 1, wherein each elastic pushing element **(140(p))** is arranged to have the central section thereof that is spaced apart from the ones of the adjacent pushing elements **(140(p))** so as to define empty spaces therebetween when the apparatus **(100)** is in the implanted configuration.

3. The apparatus **(100)** of any of the preceding claims, wherein the flexible plate member **(130)** comprises a hole **(130(h))** through which the shaft member **(120)** is inserted, allowing the flexible plate member **(130)** sliding along the shaft member **(120),** a movement of the flexible plate member **(130)** toward said first portion end (**SP(1)**) pressing the flexible extremity member **(140)** and moving said second element ends **(140(2))** toward said first portion end **(SP(1)),** thereby increasing the bending of said elastic pushing elements **(140(p)).**

4. The apparatus **(100)** of any of the preceding claims, wherein the shaft member **(120)** comprises one or more stopper elements **(120(s)),** each one configured to prevent a further sliding of the flexible plate member **(130)** away from said first portion end **(SP(1))** when the flexible plate member **(130)** is located between the first portion end **(SP(1))** and said stopper element **(120(s))** and at the same time is in contact with said flexible plate member **(130).**

5. The apparatus **(100)** of any of the preceding claims, wherein:
- the flexible extremity member **(140)** comprises a joint element **(140(j))** joining together the second element ends **(140(2))** of the elastic pushing elements **(140(p)),** and
- the joint element **(140(j))** comprises a hole **(140(h)),** the shaft member **(120)** being inserted through said hole **(140(h))** of the joint element **(140(j)),** so that said joint element **(140(j))** is able to slide along the shaft member **(120).**

6. The apparatus **(100)** of claim 5 when depending on claim 3, wherein said joint element **(140(j))** is configured to be in contact with said flexible plate member **(130),** so that a movement of the flexible plate member **(130)** toward said first portion end **(SP(1))** presses the joint element **(130)** toward said first portion end **(SP(1)).**

7. The apparatus **(100)** of any of the preceding claims, wherein said first portion end **(SP(1))** corresponds to an end **(120(2))** of the shaft member **(120).**

8. The apparatus **(100)** of any of the preceding claims, further comprising a clamp element **140(c)** configured to fix the first element ends **(140(1))** of the elastic pushing elements **(140(p))** to said first portion end **(SP(1)).**

9. The apparatus **(100)** of any of the preceding claims, in which the apparatus **(100)** comprises a thermoplastic elastomer.

10. The apparatus **(100)** of any of the preceding claims, wherein said muscle hole **(105)** is a hole located in the abdominal wall or in the pelvic floor.
